(19) Europäisches Patentamt
European Patent Office
Office européen des brevets

(11) Veröffentlichungsnummer: **0 314 005 B1**

(12) **EUROPÄISCHE PATENTSCHRIFT**

(45) Veröffentlichungstag der Patentschrift: **13.01.93**

(51) Int. Cl.[5]: **C07C 45/59**, C07C 47/277, C07C 49/255

(21) Anmeldenummer: **88117540.0**

(22) Anmeldetag: **21.10.88**

(54) **Verfahren zur Herstellung von alkoxylierten, ungesättigten Aldehyden/Ketonen.**

(30) Priorität: **30.10.87 DE 3736850**

(43) Veröffentlichungstag der Anmeldung:
**03.05.89 Patentblatt 89/18**

(45) Bekanntmachung des Hinweises auf die Patenterteilung:
**13.01.93 Patentblatt 93/02**

(84) Benannte Vertragsstaaten:
**BE DE FR GB NL**

(56) Entgegenhaltungen:
**US-A- 2 515 304**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**

EP 0 314 005 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von alkoxylierten, ungesättigten Aldehyden/Ketonen durch Umsetzung von Dialkoxytetrahydrofuranen in Gegenwart von Katalysatoren.

Bi- und trifunktionelle Verbindungen sind wertvolle Bausteine in der organischen Synthese. Besonders interessant sind Verbindungen, die einfache Schutzgruppen oder Gruppierungen mit unterschiedlicher bzw. abgestufter Reaktivität besitzen, wobei mit jeder Funktion gezielte Umsetzungen möglich sind.

Zu dieser wichtigen Stoffklasse gehören z.B. die Monoacetale des Malonaldehyds und 3-Alkoxiacroleine (Synthesis, 1985, S. 592 bis 595). Zur Herstellung solcher Verbindungen auf einem technisch gangbarem Wege scheiden Synthesen wie Ozonolyse von 1,1-Dialkoxi-3-alkenen (C.A., 1961, 55, 20926) oder die Addition von Alkoholen an Propargylaldehyd (Skoldinov et al., Zh. Org. Chim., 1968, S. 183 und 1970, S. 422) aus, da die Vorprodukte schwer zugänglich sind.

Eine weitere Möglichkeit für den Aufbau von Malonaldehydderivaten besteht in der Kombination von $C_1$-$C_2$-Bausteinen. In US-PS 2 465 586 wird die Herstellung dieser Verbindungen durch Kondensation von Ameisensäurederivaten mit Acetaldehydacetalen beschrieben, wobei die Kondensation in Gegenwart von großen Mengen an metallischem Natrium oder Natriumalkoholaten nur mit schlechten Ausbeuten gelingt.

Es wurde nun gefunden, daß man alkoxylierte, ungesättigte Aldehyde/Ketone der Formel (I)

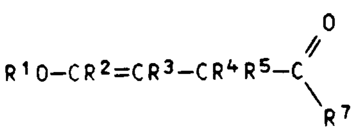

bzw.

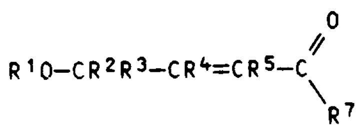

(I)

erhalten kann, indem man Dialkoxytetrahydrofurane der Formel (II)

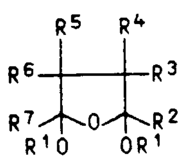

(II)

wobei die Reste $R^2$ bis $R^7$ in den Formeln (I) und (II) einander gleich oder verschieden sind und für Wasserstoff, geradkettige oder verzweigte Alkyl- oder Alkenylreste mit 1 bis 12 Kohlenstoffatomen, Cycloalkyl- oder Cycloalkenylreste mit 5 bis 8 Kohlenstoffatomen, Aryl-, Alkylaryl- oder Aralkylreste mit 6 bis 16 Kohlenstoffatomen stehen, und wobei mindestens einer der Reste von $R^3$ bis $R^6$ für Wasserstoff steht und $R^1$ Alkyl- oder Alkenylrest bedeutet, in Gegenwart von sauren, festen Heterogenkatalysatoren umsetzt.

Die Umsetzung wird bevorzugt in der Gasphase ausgeführt.

Das erfindungsgemäße Verfahren bietet einen neuen und einfachen Weg zur Herstellung von alkoxylierten, ungesättigten Aldehyden und Ketonen, in guter Ausbeute und Reinheit im kontinuierlichen Betrieb und im techischen Maßstab. Es ist überraschend, daß man bei der erfindungsgemäßen Reaktion in der Gasphase hohe Selektivitäten und Ausbeuten erzielt, da bei hohen Temperaturen mit Polymerbildung zu rechnen war.

Als Reste $R^2$ bis $R^7$ kommen geradkettige oder verzweigte Alkyl- oder Alkenylreste mit 1 bis 12, insbesondere 1 bis 8, vorzugsweise 1 bis 4 Kohlenstoffatomen in Betracht.

Alkylreste sind z.B. Methyl-, Ethyl-, Propyl-, n-Butyl-, i-Butyl-, Pentyl-, Hexyl-, Octyl- oder Decylreste. Alkenylreste sind z.B. Propenyl-, Butenyl-, Hexenyl- oder Octenylreste.

Als Cycloakylreste für $R^2$ bis $R^7$ kommen z.B. Cyclopentyl-, Cyclohexyl-, Cycloheptyl-, Cyclopentenyl- oder Cyclohexenylreste in Betracht.

Aromatische Reste für $R^2$ bis $R^7$ sind z.B. Phenyl-, Benzyl-, Toluyl-, Phenylethyl-, p-Methylbenzyl- oder p-Propylphenylreste.

Als Rest $OR^1$ kommt z.B. Methoxy-, Ethoxy-, n/i-Propoxy-, n-/i-/t-Butoxy-, Hexoxy-, Propenoxy-, Butenoxy- und Hexenoxyreste in Betracht.

Die Herstellug der Ausgangsstoffe gemäß Formel (II) ist z.B. in DE-PS 2 710 420 beschrieben.

Als Katalysatoren werden bei dem erfindungsgemäßen Verfahren saure, feste Heterogenkatalysatoren

eingesetzt. Vorteilhaft verwendet man acide zeolithische Katalysatoren. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt wrden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kukooktaeder, wodurch unterschiedlich große Hohlräume und Poren entstehen, unterscheidet man in Zeolithe vom Typ A, L, X oder Y.

Für das erfindungsgemäße Verfahren kommen als Katalysatoren in Betracht Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et. al. Elsevier Scientific Publishing Comp. 1980, S. 203 und "Crystal Structures of Ultra-stable Faujasites" Advances in Chemistry Series Nr. 101, American Chemical Society Washington, DC, S. 266ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können verschiedene chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische. Insbesondere eignen sich die Alumino-, Boro- oder Eisensilikatzeolithe des Pentasiltyps für das erfindungsgemäße Verfahren. Der Aluminosilikatzeolith kann aus einer Aluminiumverbindung, vorzugsweise $Al(OH)_3$ oder $Al_2(SO_4)$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder ohne Akali- oder Erdalkalizusatz bei 100 bis 220 °C unter autogenem Druck hergestellt werden. Hierzu gehören auch die isotaktischen Zeolithe nach EP 34 727 und EP 46 504. Die erhaltenen Aluminosilikatzeolithe enthalten je nach Wahl der Ausgangsstoffmengen ein $SiO_2/Al_2O_3$-Verhältnis von 10 bis 40.000. Derartige Aluminosilikatzeolithe kann man auch in etherischem Medium wie Diethylenglykoldimethylether, in alkoholischem Medium wie Methanol bzw. 1,4-Butandiol oder in Wasser synthetisieren.

Borosilikatzeolithe kann man bei 90 bis 200 °C unter autogenem Druck synthetisieren, indem man eine Borverbindung wie $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder ohne Alkali- oder Erdalkalizusatz zur Reaktion bringt. Hierzu gehören auch die isotaktischen Zeolithe nach EP 34 727 und EP 46 504. Die Borosiikatzeolithe können auch in etherischer Lösung, z.B. in Diethylenglykoldimethylether oder in alkoholischer Lösung, z.B. 1,6-Hexandiol hergestellt werden.

Eisensilikatzeolithe erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geqq 10$) gehören auch die sog. ZSM-Typen, Ferrierit, NU-1 un Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise bei 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochispersem $SiO_2$ und hochdispersem $Al_2O_3$, $TiO_2$, $ZrO_2$ sowie Ton. Nach der Verformung

werden die Extrudate oder Preßlinge bei 110 °C/16 h getrocknet und bei 500 °C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei man als Verstrangungs- oder Peptisierungshilfsmittel zB. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwenden kann.

Liegen die Zeolithe aufgrund der Art ihrer Herstellung nicht in der katalytisch aktiven, aciden H-Form vor, sondern z.B. in der Na-Form, dann kann diese durch Ionenaustausch mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 200 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht darin, daß man den unverformten oder verformten Zeolithen mit Metallsalzen durch einen Ionenaustausch oder durch Imprägnierung dotiert. Als Metalle werden Alkalimetalle, wie Li, Cs, K, Erdalkalimetalle, wie Mg, Ca, Sr, Metalle der 3. und 4. und 5. Hauptgruppe, wie Al, Ga, Ge, Sn, Pb, Bi, Übergangsmetalle der 4. - 8. Nebengruppe, wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe, wie Cu, Ag, Zn, seltene Erdmetalle, wie La, Ce, Pr, Nd, Fr, Yb und U eingesetzt.

Zweckmäßigerweise führt man die Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform der Zeolithe vorgenommen werden. Eine weitere Möglichkeit der Metallaufbringung auf Zeolithe ist gegeben, indem man das zeolithische Material mit einem Halogenid, Nitrat oder Oxid der beschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, etwa 30 Minuten, getränkt. Die überstehende Lösung wird am Rotationsverdampfer von Wasser befreit. Danach werden die getränkten Zeolithe bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin das reine pulverförmige Zeolithe bei 40 bis 100 °C unter Rühren etwa 24 h aufzuschlämmen. Nach Abfiltrieren, Trocknen bei etwa 150 °C und Calcinierung bei etwa 500 °C kann das zeolithische Material mit oder ohne Bindemittel zu Strängen, Pellets oder Wirbelgut weiterverarbeitet werden.

Ein Ionenaustausch der in der H-Form, Ammonium-Form oder Alkali-Form vorliegenden Zeolithe kann so vorgenommen werden, daß man die Zeolithe in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen, z.B., Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren, wie Salzsäure, Flußsäure und Phosphorsäure und/oder Wasserdampf unterwirft. Dabei geht man vorteilhaft so vor, daß man die Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolitische Material vor seiner Verformung bei erhöhter Temperatur mit 0,001 n bis 2 n, insbesondere 0,05 n bis 0,5 n Flußsäure durch Erhitzen unter Rückfluß über einen Zeitraum von 0,5 bis 5, vorzugsweise 1 bis 3 Stunden behandelt. Nach der Isolierung, z.B. durch Abfiltrieren und Auswaschen, wird das zeolithische Material zweckmäßig, bei Temperaturen von 100 bis 160 °C, getrocknet und bei Temperaturen von 450 °C bis 600 °C calciniert. Gemäß einer anderen bevorzugten Ausführungsform für die Säurebehandlung wird das zeolithische Material nach einer Verformung mit Bindemittel bei erhöhter Temperatur, zweckmäßig bei Temperaturen von 50 °C bis 90 °C, vorzugsweise 60 °C bis 80 °C, über einen Zeitraum von 0,5 bis 5, mit 12 bis 20 gew.%iger Salzsäure, behandelt. Anschließend wird das zeolithische Material ausgewaschen und bei Temperaturen von 100 bis 160 °C getrocknet und bei Temperaturen von 450 bis 600 °C calciniert. Einer HF-Behandlung kann sich auch eine HCl-Behandlung anschließen.

Nach einer anderen Arbeitsweise kann man Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger $H_3PO_4$-Lösung getränkt, bei 110 °C getrocknet und bei 500 °C calciniert.

Als Katalysatoren für das erfindungsgemäße Verfahren sind auch Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische geeignet. Als Aluminiumphosphat-Katalysatoren werden insbesondere unter hydrothermalen Bedinungen synthetisierte Aluminiumphosphate, die Zeolithstruktur besitzen, eingesetzt. Unter hydrothermalen Bedingungen hergestellte Aluminiumphosphate sind z.B. APO-5, APO-9, APO-11, APO-12, APO-14, APO-21, APO-25, APO-31 und APO-33. Synthesen dieser Verbindungen sind z.B. in EP 132 708, US 4 310 440 und US 4 473 663 beschrieben.

Beispielsweise $AlPO_4$-5 (APO-5) kann man synthetisieren, indem man Orthophosphorsäure mit Pseudoboehmit (Catapal SB®) in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert. $AlPO_4$-9 (APO-9) wird aus Orthophosphorsäure und Pseudoboehmit, in wäßriger DABCO-Lösung (1,4-Diazabicyclo-(2,2,2)-octan) bei etwa 200 °C unter autogenem Druck während 200 bis 400 h synthetisiert. Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Erfindungsgemäß werden als Siliciumaluminiumphosphate z.B. SAPO-5, SAPO-11, SAPO-31 und SAPO-34 verwendet.. Die Synthese dieser Verbindungen wird in EP-PS 103 117 und US-PS 4 440 871 beschrieben. SAPO's werden durch Kristallisation aus einer wäßrigen Mischung bei 100 bis 250 °C unter autogenem Druck während 2 h bis 2 Wochen hergestellt, wobei die Reaktionsmischung aus einer Silicium-, Aluminium- und Phosphorkomponente in wäßrigen aminoorganischen Lösungen umgesetzt wird.

SAPO-5 wird z.B. durch Mischen von $SiO_2$, suspendiert in wäßriger Tetrapropylammoniumhydroxid-Lösung, mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C .

Als Phosphatkatalysatoren kann man bei dem Verfahren auch gefällte Aluminiumphosphate einsetzen. Ein derartiges Aluminiumphosphat wird beispielsweise hergestellt, indem man 92 g Diammoniumhydrogenphosphat in 700 ml Wasser löst. Zu dieser Lösung werden 260 g $Al(NO_3)_3$ x $H_2O$ in 700 ml Wasser in 2 h zugetropft. Dabei wird der pH-Wert durch gleichzeitige Zugabe von 25 %iger $NH_3$-Lösung bei pH = 8 gehalten. Der entstandene Niederschlag wird 12 h nachgerührt, dann abgesaugt, ausgewaschen und bei 60 °C/16 h getrocknet.

Borphosphate kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, insbesondere bei 300 bis 500 °C herstellen.

Auf diese Phosphate können durch Imprägnierung, z.B. Tränken oder Aufsprühen, in manchen Fällen auch durch Ionenaustausch Modifizierungskomponenten, wie oben bei der Herstellung der Zeolithe beschrieben, aufgebracht werden. Auch hier kann eine Modifizierung mit Säuren erfolgen.

Geeignete saure Katalysatoren sind z.B. die sauer wirkenden Oxide von Elemtneenten der III. und IV. Hauptgruppe sowie der IV. bis VI. Nebengruppe des periodischen Systems, insbesondere Oxide wie Siliciumdioxid in Form von Kieselgel, Kieselgur, Qurz, weiterhin Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide. Diese Oxide können auch durch Aufbringen von Modifizieru7ngskomponenten, wie bei den Zeolithen beschrieben, dotiert werden. Auch durch Behandlung

mit Säuren ist eine Möglichkeit der Modifizierung gegeben.

Auch mit Phosphorsäure oder Borsäure getränkte Katalysatoren können verwendet werden. Phosphorsäure oder Borsäure wird z.B. auf $Si_2$-, $Al_2O_3$-, $TiO_2$- oder Bimsträger durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trockinung bzw. Calcination hergestellt werden. Die Phosphorsäure kann mit Kieselgel zusammen in einem Sprühturm versprüht werden; danach schließt sich eine Trocknung und meist eine Calcination an. Die Phosphorsäure kann aber auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die erfindungsgemäße Umwandlung wird in der Regel in der Gasphase bei 100 bis 500 ° C, insbesondere 200 bis 400 ° C, und einer Belastung WHSV = 0,1 bis 20 $h^{-1}$, insbesondere 0,5 bis 5 $h^{-1}$ g Ausgangsstoff/g Katalysator und Stunde, in einem Festbett oder in einem Wirbelbett ausgeführt.

Es ist auch möglich, die Reaktion in der Flüssigphase (Suspension-, Riesel- oder Sumpffahrweise) bei Temperaturen zwischen 50 und 200 ° C durchzuführen.

Das Verfahren kann bei Normaldruck, vermindertem oder erhöhtem Druck in diskontinuierlichem, vorzugsweise kontinuierlichem Betrieb durchgeführt werden.

Schwerflüchtige oder feste Ausgangsstoffe werden in gelöster Form, z.B. in THF-, Toluol- oder Petrolether-Lösung eingesetzt. Allgemein ist eine Verdünnung der Ausgangsstoffe mit Lösungsmitteln oder mit Inertgasen, wie $N_2$, Ar, $H_2O$-Dampf möglich. In besonderen Fällen kann auch $O_2$ verwenden.

Nach der Umsetzung werden die Produkte durch übliche Verfahren, z.B. durch Destillation aus dem Reaktionsgemisch isoliert; nichtumgesetzte Ausgangsstoffe werden gegebenenfalls in die Umsetzung zurückgeführt. Vorteilhaft werden die gasförmigen Reaktionsprodukte sofort in eine Trennung eingebracht und in ihre Einzelkomponenten zerlegt. Eine derartige Trennung kann in einer Fraktionierkolonne durchgeführt werden.

Beispiele 1 bis 5

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangssprodukte erfolgt gaschromatographisch.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8 000 g einer wäßrigen 1,6-Hexandiamin-Lösung (Mischung 50:50 Gew.% ) bei 170 ° C unter autogenem Druck in einem Rührautoklaven hergestellt. Nach Abfiltrieren und Auswaschen wird das kristalline Reaktionsprodukt bei 100 ° C/24 h getrocknet und bei 500 ° C/24 h calciniert. Dieser Borosilikatzeolith setzt sich zusammen aus 94,2 Gew.% $SiO_2$ und 2,3 Gew.% $B_2O_3$.

Katalysator A erhält man, indem man den Borosilikatzeolith mit Boehmit im Gewichtsverhältnis 60:40 zu 2-mm-Strängen verformt, bei 110 ° C/16 h trocknet und bei 500 ° C/24 h calciniert.

Katalysator B

Ein Aluminiumsilikatzeolith vom Pentasil-Typ wird unter hydrothermalen Bedingungen bei autogenem Druck und 150 ° C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 ° C/24 h getrocknet und bei 500 ° C/24 h calciniert. Dieser Aluminiumsilikatzeolith enthält 91,6 Gew.% $SiO_2$ und 4,6 Gew.% $Al_2O_3$.

Der Katalysator wird mit einem Verformungshilfsmittel zu 2-mm-Strängen verformt, bei 110 ° C/16 h trocknet und bei 500 ° C/24 h calciniert.

Katalysator C

Kommerziell erhältliches Zirkonphosphat $Zr_3(PO_4)_4$ wird in Reinsubstanz verformt.

Katalysator D

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator D enthält 8,77 Gew.% B und 28,3 Gew.% P.

Katalysator E

200 g eines im Handel erhältlichen $SiO_2$ (D 11-10®) werden mit 600 ml 15 %iger HCl bei 80 °C/1 h behandelt. Danach wird das Material Cl-frei gewaschen, bei 110 °C getrocknet und bei 600 °C/1 h calciniert.

Die mit diesen Katalysatoren erzielten Versuchsergebnisse und Versuchsbedingungen sind in der folgenden Tabelle zusammengefaßt.

Tabelle: Umsetzung von 2,5-Dimethoxytetrahydrofuran zu 4-Methoxy-butenal Formel (I)

| Beispiel | 1 | 2 | 3 | 4 | 5 |
|---|---|---|---|---|---|
| Katalysator | A | B | C | D | E |
| Temperatur °C | 300 | 300 | 300 | 300 | 300 |
| WHSV $h^{-1}$ | 3 | 3 | 3 | 3 | 3 |
| Umsatz % | 77,2 | 30,5 | 85,5 | 49,4 | 94,7 |
| Selektivität % | 67,3 | 75,0 | 93,2 | 75,9 | 91,4 |

Nebenprodukte der Reaktion sind Furan, 2-Methoxydihydrofuran, Butanaldimethylacetal und Acetale der Aldehydverbindungen.

**Patentansprüche**

**1.** Verfahren zur Herstellung von alkoxylierten, ungesättigten Aldehyde/Ketone der Formel (I)

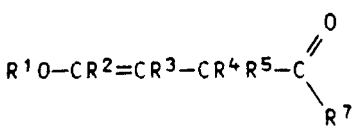 bzw. 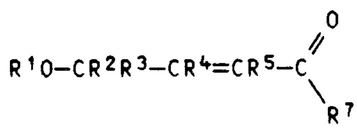 (I)

dadurch gekennzeichnet, daß man Dialkoxytetrahydrofurane der Formel (II)

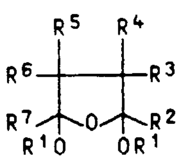 (II)

wobei die Reste $R^2$ bis $R^7$ in den Formeln (I) und (II) einander gleich oder verschieden sind und für Wasserstoff, geradkettige oder verzweigte Alkyl- oder Alkenylreste mit 1 bis 12 Kohlenstoffatomen, Cycloalkyl- oder Cycloalkenylreste mit 5 bis 8 Kohlenstoffatomen, Aryl-, Alkylaryl- oder Aralkylreste mit 6 bis 16 Kohlenstoffatomen stehen, und wobei mindestens einer der Reste von $R^3$ bis $R^6$ für Wasserstoff steht und $R^1$ Alkyl- oder Alkenylrest bedeutet, in Gegenwart von sauren, festen Heterogenkatalysatoren umsetzt.

**2.** Verfahren nach Anspruch 1, dadurch gekennzeichnet, daß man 2,5-Dimethoxytetrahydrofuran umsetzt.

**3.** Verfahren nach Anspruch 1 und 2, dadurch gekennzeichnet, daß man als Heterogenkatalysatoren Zeolithe verwendet.

**4.** Verfahren nach Anspruch 1 bis 3, dadurch gekennzeichnet, daß man als Katalysatoren Zeolithe des Pentasiltyps, insbesondere Aluminiumsilikatzeolithe, Borosilikatzeolithe und/oder Eisensilikatzeolithe verwendet.

**5.** Verfahren nach Anspruch 1 bis 3, dadurch gekennzeichnet, daß man als Katalysatoren Zeolithe des Faujasittyps verwendet.

**6.** Verfahren nach Anspruch 1 bis 5, dadurch gekennzeichnet, daß man mit Alkalimetallen, Übergangsmetallen, Seltenen Erdmetallen dotierte Zeolithe als Katalysatoren verwendet.

**7.** Verfahren nach Anspruch 1 und 2, dadurch gekennzeichnet, daß man als Heterogenkatalysatoren Phosphate verwendet.

**8.** Verfahren nach Anspruch 1, 2 und 7, dadurch gekennzeichnet, daß man als Heterogenkatalysatoren Phosphate der Elemente B, Al, Zr, Ce, Fe, Sr oder deren Gemische verwendet.

**9.** Verfahren nach Anspruch 1, 2 und 7, dadurch gekennzeichnet, daß man als Katalysatoren hydrothermal hergestellte Aluminiumphosphate, Siliciumaluminiumphosphate, Siliciumeisenaluminiumphosphate und/oder Boraluminiumphosphate verwendet.

**10.** Verfahren nach Anspruch 1 und 2, dadurch gekennzeichnet, daß man als Heterogenkatalysatoren Metalloxide der Elemente wie Si, Al, Ti, Zr, B, Fe, W, Mo, Nb, V verwendet.

**11.** Verfahren nach Anspruch 1, 2 und 10, dadurch gekennzeichnet, daß man als Katalysatoren mit Säuren behandelte Metalloxide verwendet.

**12.** Verfahren nach Anspruch 1 und 2, dadurch gekennzeichnet, daß man als Katalysatoren Phosphorsäure bzw. Borsäure auf Trägermaterialien verwendet.

**13.** Verfahren nach Anspruch 1 bis 12, dadurch gekennzeichnet, daß man die Reaktion in der Gasphase durchführt.

**Claims**

**1.** A process for preparing alkoxylated, unsaturated aldehydes/ketones of the formula (I)

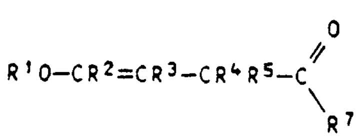 or 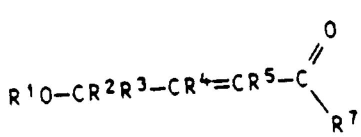 (I)

which comprises reacting dialkoxytetrahydrofurans of the formula (II)

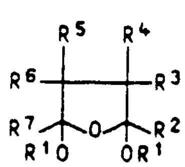

(II)

where the radicals $R^2$ to $R^7$ in the formulae (I) and (II) are identical or different and each is hydrogen, straight-chain or branched alkyl or alkenyl of from 1 to 12 carbon atoms, cycloalkyl or cycloalkenyl of

from 5 to 8 carbon atoms, or aryl, alkylaryl or aralkyl of from 6 to 16 carbon atoms and at least one of the radicals $R^3$ to $R^6$ is hydrogen and $R^1$ is alkyl or alkenyl, in the presence of acidic, solid heterogeneous catalysts.

**2.** A process as claimed in claim 1, wherein 2,5-dimethoxytetrahydrofuran is reacted.

**3.** A process as claimed in claim 1 or 2, wherein the heterogeneous catalysts used are zeolites.

**4.** A process as claimed in any of claims 1 to 3, wherein the catalysts used are zeolites of the pentasil type, in particular aluminum silicate zeolites, borosilicate zeolites and/or iron silicate zeolites.

**5.** A process as claimed in any of claims 1 to 3, wherein the catalysts used are zeolites of the faujasite type.

**6.** A process as claimed in any of claims 1 to 5, wherein the catalysts used are zeolites doped with alkali metals, transition metals or rare earth metals.

**7.** A process as claimed in claim 1 or 2, wherein the heterogeneous catalysts used are phosphates.

**8.** A process as claimed in any of claims 1, 2 and 7, wherein the heterogeneous catalysts used are phosphates of the elements B, Al, Zr, Ce, Fe, Sr or mixtures thereof.

**9.** A process as claimed in any of claims 1, 2 and 7, wherein the catalysts used are hydrothermally synthesized aluminum phosphates, silicon aluminum phosphates, silicon iron aluminum phosphates and/or boron aluminum phosphates.

**10.** A process as claimed in claim 1 or 2, wherein the heterogeneous catalysts used are metal oxides of elements such as Si, Al, Ti, Zr, B, Fe, W, Mo, Nb, V.

**11.** A process as claimed in any of claims 1, 2 and 10, wherein the catalysts used are acid treated metal oxides.

**12.** A process as claimed in claim 1 or 2, wherein the catalysts used are phosphoric acid or boric acid on carrier materials.

**13.** A process as claimed in any of claims 1 to 12, wherein the reaction is carried out in the gas phase.

**Revendications**

**1.** Procédé de préparation d'aldébydes/cétones alcoxylés insaturés de formule (I)

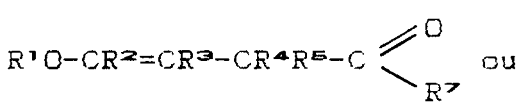 ou 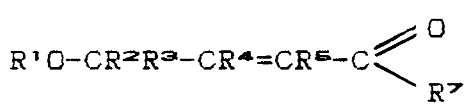 (I)

caractérisé en ce qu'on fait réagir, en présence de catalyseurs hétérogènes acides solides, des dialcoxytétrahydrofurannes de formule (II)

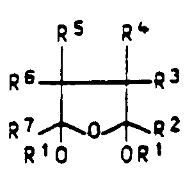 (II)

les restes $R^2$ à $R^7$ dans les formules (I) et (II) étant identiques ou différents les uns des autres et étant mis pour des atomes d'hydrogène, pour des restes alkyle ou alcényle à chaîne droite ou ramifiée à 1-12 atomes de carbone, pour des restes cycloalkyle ou cycloalcényle à 5-8 atomes de carbone ou pour des restes aryle, alkylaryle ou aralkyle a 6-16 atomes de carbone, l'un au moins des restes $R^3$ à $R^6$ étant mis pour un atome d'hydrogène, et $R^1$ representant un reste alkyle ou alcényle.

**2.** Procédé selon la revendication 1, caractérisé en ce qu'on fait reagir du 2,5-diméthoxytétrahydrofuranne.

**3.** Procédé selon la revendication 1 ou 2, caractérisé en ce qu'on utilise des zéolithes comme catalyseurs hétérogènes.

**4.** Procédé selon l'une quelconque des revendications 1 à 3, caractérisé en ce qu'on utilise, comme catalyseurs, des zéolithes du type pentasil, en particulier des zéolithes d'aluminosilicate, des zéolithes de borosilicate et/ou des zéolithes de ferrosilicate.

**5.** Procédé selon l'une quelconque des revendications 1 à 3, caractérisé en ce qu on utilise, comme catalyseurs, des zéolithes du type faujasite.

**6.** Procédé selon l'une quelconque des revendications 1 à 5, caractérisé en ce qu'on utilise, comme catalyseurs, des zéolithes dopées par des métaux alcalins, des métaux de transition ou des métaux des terres rares.

**7.** Procédé selon la revendication 1 ou 2, caractérisé en ce qu'on utilise des phosphates comme catalyseurs hétérogènes.

**8.** Procédé selon les revendications 1, 2 et 7, caractérisé en ce qu'on utilise, comme catalyseurs hétérogènes, des phosphates des éléments B, Al, Zr, Ce, Fe, Sr ou des mélanges de ceux-ci.

**9.** Procédé selon les revendications 1, 2 et 7, caractérise en ce qu'on utilise, comme catalyseurs, des phosphates d'aluminium, des phosphates de silicium-aluminium, des phosphates de silicium-fer-aluminium et/ou des phosphates de bore-aluminium préparés par synthèse hydrothermale.

**10.** Procédé selon la revendication 1 ou 2, caractérisé en ce qu'on utilise, comme catalyseurs hétérogènes, des oxydes métalliques des éléments tels que Si, Al, Ti, Zr, B, Fe, W, Mo, Nb, V.

**11.** Procédé selon les revendications 1, 2 et 10, caractérisé en ce qu'on utilise, comme catalyseurs, des oxydes métalliques traités par des acides

**12.** Procédé selon la revendication 1 ou 2, caractérisé en ce qu'on utilise, comme catalyseurs, de l'acide phosphorique ou de l'acide borique sur des matières de support.

**13.** Procédé selon l'une queconque des revendications 1 à 12, caractérisé en ce qu'on mène la réaction en phase gazeuse.